# EUROPÄISCHE PATENTSCHRIFT

(11) **EP 0 770 675 B1**
(45) Veröffentlichungstag und Bekanntmachung des Hinweises auf die Patenterteilung: **14.01.1998**
(21) Anmeldenummer: 95810560.3
(22) Anmeldetag: 11.09.1995
(51) Int. Cl.: C12M 1/107, C12M 1/02

(54) **Fermenter für anaerobe Fermentation**
Fermenter for anaerobic fermentation
Fermenteur pour fermentation anaérobie

(43) Veröffentlichungstag der Anmeldung: 02.05.1997
(73) Patentinhaber: Schmid, Walter, CH-8152 Opfikon (CH)
(72) Erfinder: Schmid, Walter, CH-8152 Opfikon (CH); Huwiler, Theo, CH-8954 Geroldswil (CH)
(74) Vertreter: Feldmann, Clarence Paul

(56) Entgegenhaltungen:
- DE-A- 3 151 187
- DE-A- 3 226 607
- DE-A- 3 243 103
- DE-A- 3 326 607
- DE-C- 923 539
- FR-A- 1 094 014
- FR-A- 1 179 296
- FR-A- 2 471 124

## Beschreibung

Die vorliegende Erfindung betrifft einen pfropfstrombetriebenen Fermenter gemäss Oberbegriff des Patentanspruches 1.

Derartige Fermenter dienen vorallem zur Vergärung von biogenen Siedlungsabfällen für die Gewinnung von Biogas. Der längsgeformte Vergärungsbehälter des Fermenters wird dabei bevorzugterweise über einen stirnseitigen Einlass mit dem zu vergärenden Material beschickt. Das Material wird im Pfropfstrombetrieb durch den Fermenter gefördert und verlässt diesen über einen wiederum stirnseitigen Auslass.
Aus EP-A-0'621'336 ist bekannt, den Fermenter liegend anzuordnen, wobei das Faulgut mit einem Rührwerk durchmischt wird. Um eine optimale Reaktionstemperatur zu erhalten, wird der Fermenter zusätzlich beheizt. Dabei ist darauf zu achten, dass die Temperatur im Fermenter nicht zu tief liegt, da sonst die Vergärung zu langsam verläuft, sie darf jedoch auch nicht zu hoch sein, da sonst die für die Vergärung notwendigen Methanbakterien zerstört werden. Eine optimale Reaktionstemperatur liegt zwischen 55-60°C.

Die Abmessungen des Vergärungsbehälters des Fermenters können variieren. Typische Vergärungsbehälter sind zylinderförmig gestaltet, weisen ein Volumenfassungsvermögen von bis zu 600 m³ auf und erzielen einen Durchsatz an zu vergärendem Material von bis zu 8000 t/Jahr.
Das Grundmaterial der bekannten Vergärungsbehälter besteht stets aus Stahl. Deshalb sind nur die kleineren Fermenter bis zu einem Volumen von 360 m³ einstückig geformt, grössere Fermenter müssen vor Ort sektionsweise zusammengesetzt werden.

Die als Fermenter dienenden Stahlbehälter weisen mehrere Nachteile auf:
Da sie relativ gross sind, sind sie nur schwer transportierbar. Grosse Behälter müssen zudem vor Ort zusammengeschweisst werden, was oft durch Platzmangel erschwert wird.
Der Stahlfermenter muss mit einem Betongebäude umgeben werden, damit er vor äusseren Einwirkungen wie Korrosion geschützt ist. Dadurch werden die Kosten für die Erstellung derartiger Vergärungsanlagen erhöht.

Die Regelung der Reaktionstemperatur ist bei Stahlfermentern relativ schwierig, da sie wegen den thermischen Materialeigenschaften ein schnell reagierendes thermisches System bilden. In der Fermentation werden jedoch träge thermische Systeme bevorzugt, da dies die elektronische Regelung der Heizung vereinfacht.

Ferner ist die Integration einer Heizung in einen Stahlfermenter schwierig. Deshalb werden im allgemeinen Kupferrohre um den äusseren Mantels des Stahlfermenters gewickelt und anschliessend von einer Isolationsschicht umgeben. Auch dies erhöht wiederum die Herstellungskosten und erschwert die Wartung des Fermenters.

FR-A-2'471'124 offenbart ferner einen liegenden Fermenter, welcher aus Metall, aus Eisenbeton oder aus Kunststoff gefertigt sein kann.

Es ist deshalb Aufgabe der Erfindung, einen Fermenter zur anaeroben Vergärung zu schaffen, der die obengenannten Nachteile behebt.

Diese Aufgabe löst ein Fermenter mit den Merkmalen des Patentanspruches 1. In Anspruch 9 wird ein Verfahren zur Erstellung eines derartigen Fermenters beansprucht.

Der erfindungsgemässe Fermenter weist einen Vergärungsbehälter aus Beton auf anstatt aus Stahl. Dadurch kann er vor Ort und in jeder beliebigen Grösse hergestellt werden.
Da der Beton selber eine genügende Festigkeit aufweist und gegen Umwelteinflüsse weitgehend resistent ist beziehungsweise mit einfachen, üblichen Mitteln geschützt werden kann, erübrigt sich eine weitere abschirmende Hülle in Form eines Betongehäuses. Dadurch ist es neu auch möglich, den Fermenter direkt, ohne zusätzliches Mauerwerk, in das Erdreich ganz oder teilweise einzugraben. In dieser Variante empfiehlt es sich, eine Kälte- und gegen Wasser resistente Isolationsschicht zwischen Beton und Erde vorzusehen.
Der Fermenter kann nun typische Grössen bis zu 1500 m³ mit einem Jahresdurchsatz von 20'000 t aufweisen.
Der Betonmantel kann beliebig dick ausgestaltet sein. Zudem weist Beton einen kleinen Wärmeleitkoeffizienten auf, so dass der Fermenter ein träges thermisches System bildet. Der Vergärungsbehälter aus Beton dient als Wärmespeicher, der für eine ausgeglichene Temperatur im Innern des Fermenters sorgt. Die Heizung kann ähnlich den bekannten Bodenheizungen in Wohnräumen in den Betonmantel eingebaut werden. Dabei können anstelle der Kupferrohre kostengünstigere Kunststoffrohre verwendet werden.
Ein weiterer Vorteil ist, dass die mit dem Fermenter gekoppelte Service-Infrastruktur, wie Kontroll- und Bedienungsgang, anschliessend und einstückig am Fermenter angebaut werden kann.

Der Fermenter weist eine Innenverkleidung aus Stahl auf, die bei der Erstellung des Fermenters als verlorene Schalung gedient hat. Dies hat den Vorteil, dass die Innenfläche des Beton-Vergärungsbehälters vor Säuren, die bei der Vergärung entstehen, geschützt ist. Zudem gewährleistet die vorgängig als Verschalung dienende Innenverkleidung die Dichtheit des Vergärungsbehälters, so dass der Beton nicht mehr vorgespannt werden muss und eine hohe Kunststoffvergütung in diesem Falle entbehrlich ist.

In den beiliegenden Zeichnungen sind Ausführungsbeispiele des Erfindungsgegenstandes dargestellt, die in der nachfolgenden Beschreibung erläutert werden. Es zeigen
- Figur 1: einen Querschnitt durch einen Fermenter senkrecht zur Förderrichtung;
- Figur 2: einen Längsschnitt durch den Fermenter;
- Figur 3: einen Längsschnitt durch eine weitere Ausführungsform des Fermenters;
- Figur 4: einen Querschnitt durch einen Doppelfermenter und
- Figur 5: einen Querschnitt durch eine weitere Ausführungsform des Fermenters.

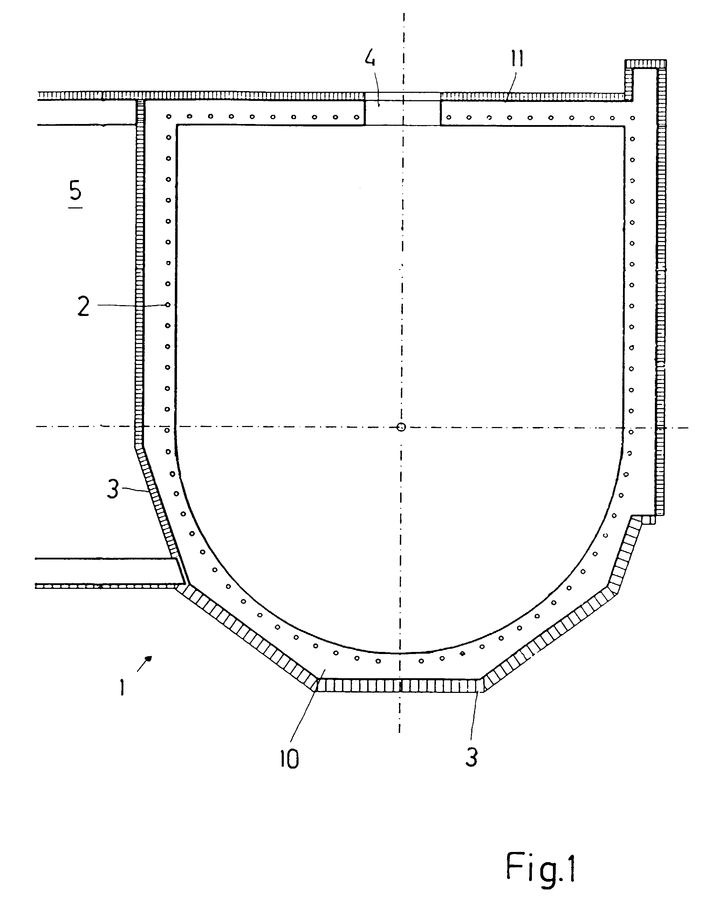

Der erfindungsgemässe Fermenter weist einen Vergärungsbehälter 1 aus Beton auf. Der Vergärungsbehälter 1 weist bevorzugterweise einen u-förmigen Querschnitt auf, gebildet durch einen Mantel 10 und einen die zwei Schenkel des U's abschliessend verbindenden Deckel 11. Dabei ist der Querschnitt senkrecht zur Förderrichtung des zu fermentierenden Gutes u-förmig, wie in Figur 1 ersichtlich. Typische Masse für einen Fermenter mit einem Volumenfassungsvermögen von ungefähr 1'000 m³ sind für die Länge 32 m, für die Höhe 6,3 m und für die Wandstärke 0.4 m. In Figur 1 ist teilweise ein angebauter, begehbarer Kontroll- und Bedienungsgang 5 sichtbar, der für die Bedienung des Fermenters und zur Kontrolle des Vergärungsprozesses dient.

Der Innenraum I des Fermenters wird über einen stirnseitigen Einlass mit dem zu vergärenden Material gefüllt, das im Pfropfstrombetrieb bis zum stirnseitigen Auslass gefördert wird. Oben im Deckel 11 ist eine Absaugöffnung 4 für die Biogasentnahme vorhanden. Diese Absaugöffnung 4 ist in diesem Beispiel ungefähr in der Mitte der Längsausdehnung des Betonbehälters 1 angeordnet. Der Vergärungsbehälter kann aus kunststoffvergütetem Beton bestehen, damit er durch die bei der Vergärung entstehenden Säuren nicht angegriffen wird. In diesem Fall ist der Vergärungsbehälter bevorzugterweise aus vorgespanntem Beton gefertigt, um die Dichtheit des Fermenters zu gewährleisten.

Da der Vergärungsbehälter 1 aus Beton stabil ist, kann er direkt erdverlegt werden, wobei der Deckel 11 begehbar ist. In diesem Fall wird der Vergärungsbehälter 1 von einer Isolationsschicht 3 umgeben, die kälteisolierend und/oder gegen Wasser resistent ist.

Der hier dargestellte Fermenter ist waagrecht liegend angeordnet und weist in seinem Innern ein hier nicht dargestelltes Rührwerk auf. Andere liegende, geneigte Anordnungen des Fermenters sind moglich.

Innerhalb der Wandung des Vergärungsbehälters 1 ist die Heizung zur Steuerung der Reaktionstemperatur verlegt. Die einzelnen Heizelemente sind in der Figur 1 mit der Bezugsziffer 2 bezeichnet. Sie bestehen hier aus schlaufenförmigen Rohren, bevorzugterweise aus Kunststoff. Diese Heizelemente 2 sind näher zum Innenraum I als zur Aussenseite des Fermenters hin angeordnet, so dass sie eine bessere Wärmeübertragung zum Innenraum ermöglichen.

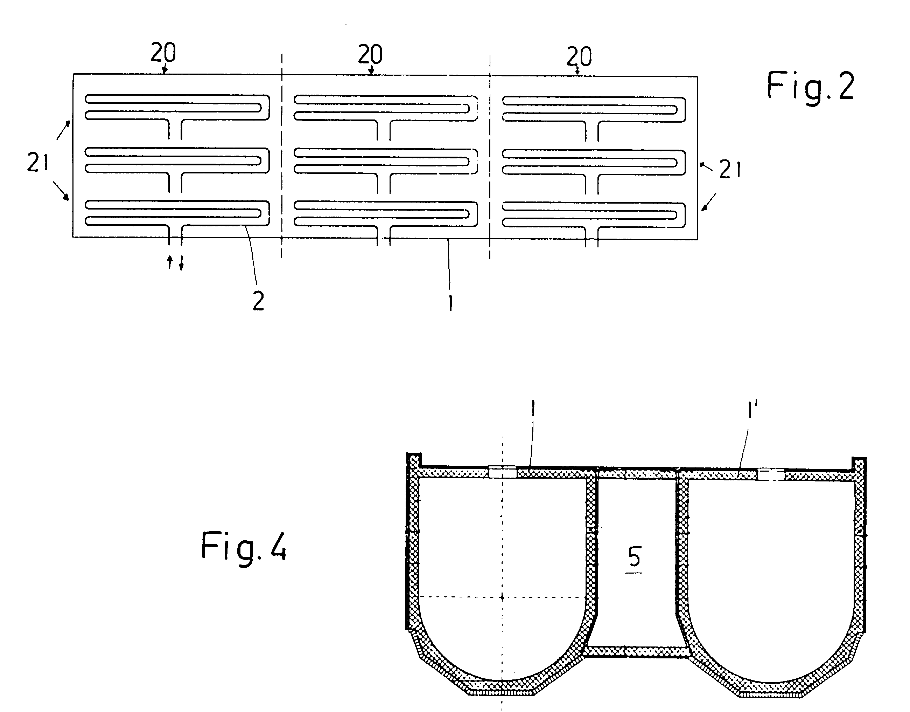

In Figur 2 ist eine bevorzugte Anordnung der Heizelemente 2 dargestellt. Die schlaufenförmigen Rohre bilden entlang der Förderrichtung des organischen Materiales voneinander unabhängige Abschnitte, sogenannte Sektionen 20. Dadurch kann an verschiedenen Stellen des Vergärungsbehälters 1 auf verschiedene Temperaturen geregelt werden. So ist beispielsweise am vorderen Ende des Fermenters eine höhere Heizleistung erforderlich, damit der Vergärungsprozess gestartet werden kann. Im hinteren Bereich des Fermenters genügt jedoch eine kleinere Heizleistung.

Zudem sind die einzelnen von den Heizelementen 2 gebildeten Sektionen 20 in der zur Förder- oder Längsrichtung senkrechten Ebene in voneinander unabhängige Abschnitte unterteilt, in sogenannte Register 21, die übereinander angeordnet sind. Fällt ein Heizelement 2 aus, so wird die entsprechende Sektion trotzdem noch durch die übrigen Heizelemente 2 beheizt. Dadurch wird, da die Heizelemente 2 durch diese Anordnung kleinere Abmessungen aufweisen, der Betrieb des Fermenters nicht in Frage gestellt.
In einer bevorzugten Ausführungsform sind pro Sektion im Bodenbereich des Fermenters zwei Heizelemente vorhanden, beidseitig der Flanke des Fermentes ist je ein Heizelement und auf der Oberseite ebenfalls ein Heizelement angeordnet, so dass pro Sektion insgesamt fünf Heizelemente eingesetzt werden.

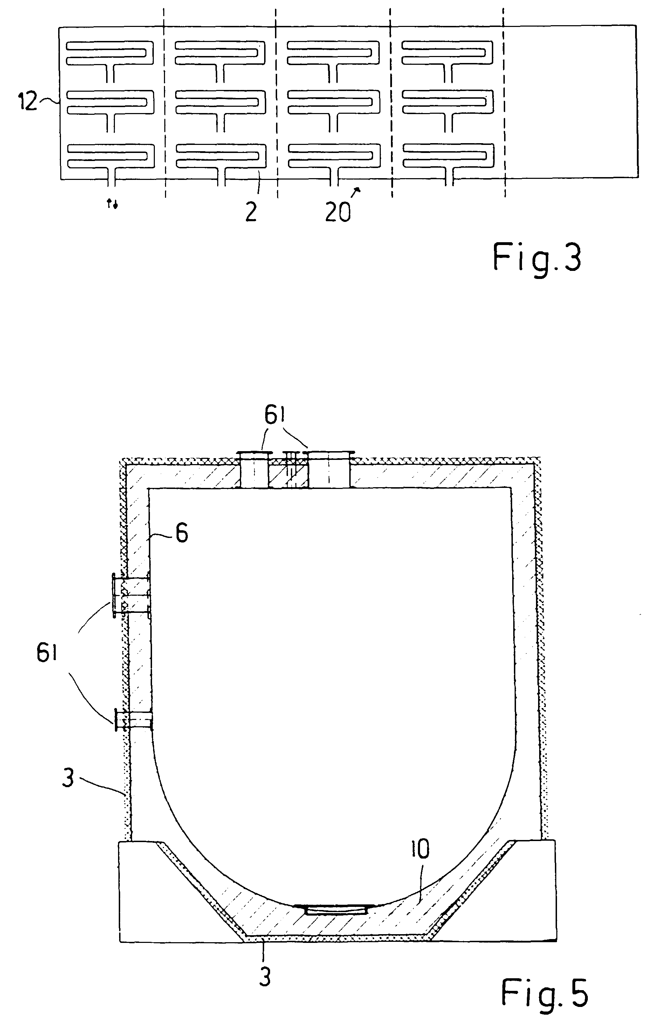

In Figur 3 ist eine weitere Ausführunsform des Fermenters mit einer anderen Heizung dargestellt. Die hier dargestellte Heizung weist vier Sektionen 20 auf. Dabei sind im ausgangsseitigen Bereich des Vergärungsbehälters keine Heizelemente 2 vorhanden. Hingegen ist die eingangsseitige Stirnseite 12 des Vergärungsbehälters ebenfalls beheizt.

In Figur 4 ist eine weitere Ausführungsform des erfindungsgemässen Fermenters dargestellt. Es handelt sich hierbei um einen Doppelfermenter. Dieser besteht aus zwei wie oben beschriebenen Vergärungsbehälters 1, 1' aus Beton, die entlang ihrer Förder- und Längsrichtung über einen Kontroll- und Bedienungskanal 5 miteinander verbunden sind, der ebenfalls aus Beton gebaut ist. Diese Ausführungsform hat den Vorteil, dass auf relativ geringem Platz zwei getrennte Fermenter mittels derselben Infrastruktur bedient und überwacht werden können. Der Betonaufbau erlaubt eine Bauweise, die der entsprechenden Umgebung und dem ortsbedingten Mengenbedarf an zu entsorgenden biogenen Abfällen angepasst ist.

In der Figur 5 ist die Innenverkleidung 6 aus Stahl des erfindungsgemässen Fermenters dargestellt.
Bei der Betonierung des Vergärungsbehälters wird eine verlorene Schalung aus Stahl verwendet, die als Innenverkleidung 6 im Vergärungsbehälter 1 verbleibt. Sie dient dem erstellten Vergärungsbehälter 1 als Schutz gegen Säuren, so dass sich eine weitere Beschichtung erübrigt. Diese Innenverkleidung ist mechanisch stärker belastbar als eine Kunststoffbeschichtung.
Bevorzugterweise weist die Stahlverschalung eine Dicke von ungefähr 4 mm auf.
Dank dieser verlorenen Stahlschalung kann der Vergärungsbehälter abschnittsweise betoniert werden, wobei der Beton nicht vorgespannt werden muss. Die Stahlschalungen der einzelnen Abschnitte werden anschliessend miteinander verschweisst, so dass sie die Dichtheit des Vergärungsbehälters gewährleisten. Der Schweissvorgang wird durch die dünne Wandung der Stahlschalung vereinfacht. Die verschiedenen Anschlussarmaturen 61 des Fermenters, wie Luken, Durchführungen, Lager und dergleichen werden ebenfalls an die Stahlschalung angeschweisst.

Die Stahlwandung der Schalung kann relativ dünn sein, da sie während dem Betrieb des Fermenters keine statische Funktion mehr besitzt. Besonders vorteilhaft ist aber auch die Möglichkeit, bereits in der verlorenen Schalung die erforderlichen Anschlussarmaturen anzubringen.

## Patentansprüche

1. Pfropfstrombetriebener, liegender Fermenter zur anaeroben Fermentation von organischen Materialien mit einem Einlass, einem Auslass, einem Rührwerk und einer Heizung (2) zur Steuerung der Reaktionstemperatur, wobei der Fermenter einen Vergärungsbehälter (1) aus Beton aufweist,
dadurch gekennzeichnet, dass der Vergärungsbehälter (1) eine Innenverkleidung (6) aus Stahl aufweist, wobei diese Innenverkleidung (6) eine verlorene Schalung zur Erstellung des Beton-Vergärungsbehälters (1) ist und
dass die Heizung (2) innerhalb der Wandung des Vergärungsbehälters (1) angeordnet ist.

2. Fermenter nach Anspruch 1, dadurch gekennzeichnet, dass die Innenverkleidung (6) eine Dicke von annähernd 4 mm aufweist.

3. Fermenter nach Anspruch 1, dadurch gekennzeichnet, dass er senkrecht zur Förderrichtung einen u-förmigen Querschnitt aufweist.

4. Fermenter nach Anspruch 1, dadurch gekennzeichnet, dass die Heizung aus einzelnen schlaufenförmigen Heizelementen (2) besteht.

5. Fermenter nach Anspruch 4, dadurch gekennezeichnet, dass die Heizelemente (2) näher zum Innenraum (I) als zur Aussenseite des Vergärungsbehälters (1) hin angeordnet sind.

6. Fermenter nach Anspruch 5, dadurch gekennzeichnet, dass die Heizelemente (2) entlang der Förderrichtung des organischen Materiales in voneinander unabhängige Abschnitte, nämlich Sektionen (20), unterteilt sind.

7. Fermenter nach Anspruch 6, dadurch gekennzeichnet, dass die Heizelemente (2) der Sektionen (20) in der zur Förderrichtung des organischen Materiales senkrechten Ebene in voneinander unabhängige Abschnitte, nämlich Register (21), unterteilt sind.

8. Vergärungsanlage mit zwei Fermentern gemäss Anspruch 1, dadurch gekennzeichnet, dass die Vergärungsbehälter (1) mittels einem begehbaren Kontroll- und Bedienungskanal (5) miteinander verbunden sind.

9. Verfahren zur Erstellung eines Fermenters nach Anspruch 1, dadurch gekennzeichnet, dass eine verlorene Schalung aus Stahl verwendet wird, dass der Vergärungsbehälter abschnittsweise betoniert wird und dass die verlorene Schalung der einzelnen Abschnitte miteinander verschweisst werden.

10. Verfahren nach Anspruch 9, dadurch gekennzeichnet, dass in die verlorene Schalung Anschlussarmaturen (61) integriert werden.

## Claims

1. A plug flow-operated, horizontal fermenter for the anaerobic fermentation of organic materials, with an inlet, an outlet, a stirrer and a heating means (2) for controlling the reaction temperature, the fermenter having a concrete fermentation tank (1), characterised in that the fermentation tank (1) has a steel inner lining (6), which is a permanent shuttering for the production of the concrete fermentation tank (1), and that the heating means (2) is located within the wall of the fermentation tank (1).

2. A fermenter according to claim 1, characterised in that the inner lining (6) has a thickness of approximately 4 mm.

3. A fermenter according to claim 1, characterised in that it has a U-shaped cross-section perpendicular to the direction of feed.

4. A fermenter according to claim 1, characterised in that the heating means comprises individual, loop-like heating elements (2).

5. A fermenter according to claim 4, characterised in that the heating elements (2) are closer to the interior (I) than to the exterior of the fermentation tank (1).

6. A fermenter according to claim 5, characterised in that the heating elements (2) are subdivided along the direction of feed of organic material into independent zones, namely sections (20).

7. A fermenter according to claim 6, characterised in that the heating elements (2) of the sections (20) are subdivided in the plane perpendicular to the direction of feed of organic material into independent zones, namely registers (21).

8. A fermentation plant with two fermenters according to claim 1, characterised in that the fermentation tanks (1) are interconnected by means of a walk-through operating and control pipe (5).

9. A method for the production of a fermenter according to claim 1, characterised in that a steel permanent shuttering is used, that the fermentation tank is zonally concreted and that the individual permanent shuttering zones are welded together.

10. A method according to claim 9, characterised in that connection fittings (61) are integrated into the permanent shuttering.

## Revendications

1. Fermenteur couché, à fonctionnement en régime bouchon, pour fermentation anaérobie de matières organiques avec une entrée, une sortie, un agitateur et un chauffage (2) pour le réglage de la température de réaction, tandis que le fermenteur comporte un récipient de fermentation (1) en béton, caractérisé en ce que le récipient de fermentation (1) présente un chemisage intérieur (6) en acier, ce chemisage intérieur (6) étant un coffrage perdu servant à la préparation du récipient de fermentation en béton (1) et que le chauffage (2) est disposé à l'intérieur de la paroi du récipient de fermentation (1).

2. Fermenteur selon la revendication 1, caractérisé en ce que le chemisage intérieur (6) présente une épaisseur d'environ 4 mm.

3. Fermenteur selon la revendication 1, caractérisé en ce qu'il présente perpendiculairement à la direction d'alimentation une section transversale en forme de U.

4. Fermenteur selon la revendication 1, caractérisé en ce que le chauffage consiste en des éléments chauffants (2) individuels en forme de boucles.

5. Fermenteur selon la revendication 4, caractérisé en ce que les éléments chauffants (2) sont disposés plus près de l'espace intérieur (I) que de la face externe du récipient de fermentation (1).

6. Fermenteur selon la revendication 5, caractérisé en ce que les éléments chauffants (2) sont subdivisés le long de la direction d'alimentation de la matière organique en tronçons indépendants les uns des autres, à savoir des sections (20).

7. Fermenteur selon la revendication 6, caractérisé en ce que les éléments chauffants (2) des sections (20) sont subdivisés dans le plan perpendiculaire à la direction d'alimentation de la matière organique en des tronçons indépendants les uns des autres, à savoir des registres (21).

8. Installation de fermentation comportant deux fermenteurs selon la revendication 1, caractérisée en ce que les récipients de fermentation (1) sont reliés entre eux par un passage de contrôle et de service (5) praticable.

9. Procédé pour la réalisation d'un fermenteur selon la revendication 1, caractérisé en ce qu'on emploie un coffrage perdu en acier, que le récipient de fermentation est bétoné par tronçons et que les coffrages perdus des tronçons individuels sont soudés entre eux.

10. Procédé selon la revendication 9, caractérisé en ce que dans le coffrage perdu sont intégrées des armatures d'assemblage (61).
